# EUROPEAN PATENT APPLICATION

(11) **EP 4 742 170 A1**
(43) Date of publication of application: **13.05.2026**
(21) Application number: 24306884.8
(22) Date of filing: 08.11.2024
(51) Int. Cl.: G06T 15/08, G16H 30/00, G06T 5/00, G06T 19/00

(54) **GENERATING A 2D IMAGE OF AN ANATOMICAL STRUCTURE FROM 3D MEDICAL DATA**

(71) Applicant: Koninklijke Philips N.V., 5656 AG Eindhoven (NL)
(72) Inventor: MORY, Benoit, 5656 Eindhoven (NL); SOMPHONE, Oudom, 5656 Eindhoven (NL); ATTIA, Emmanuel, 5656 Eindhoven (NL); FRADKIN, Maxim, 5656 Eindhoven (NL)
(74) Representative: Philips Intellectual Property & Standards

(57) **Abstract**

The present disclosure provides concepts for processing pixels of interest of a 3D volume of an anatomical structure to generate a 2D image of the anatomical structure. The method includes, for each of the pixels of interest: identifying one or more compound pixels in the vicinity of the respective pixel in the 3D volume based on one or more features of the anatomical structure included in the 3D volume; and determining an updated value of the respective pixel based on values of the compound pixels. The method further includes generating the 2D image based on the updated values of the pixels of interest. The method allows for adaptive compounding based on the shape of the anatomical structure included in the 3D volume, such that blurring and ghosting artifacts are reduced, while maintaining the benefits of compounding techniques (e.g., noise reduction).

## Description

### FIELD OF INVENTION

The present invention relates to medical image processing, and more particularly to systems and methods for generating two-dimensional (2D) images from three-dimensional (3D) volumetric data of anatomical structures using adaptive compounding techniques.

### BACKGROUND OF INVENTION

Three-dimensional (3D) medical imaging technologies, such as computed tomography (CT), magnetic resonance imaging (MRI), and ultrasound, have revolutionized the field of medical diagnostics. These modalities provide detailed volumetric data of anatomical structures, allowing for comprehensive analysis and visualization. However, clinicians often need to examine specific two-dimensional (2D) slices or cross-sections of the 3D volume for diagnosis, treatment planning, and patient communication.

Extracting 2D images from 3D volumetric data presents several challenges. Standard techniques for generating 2D slices, such as multi-planar reconstruction (MPR), often result in images with reduced quality compared to the original 3D data. Noise, artifacts, and loss of detail can occur during the extraction process, potentially impacting diagnostic accuracy. Additionally, when the desired 2D plane does not align with the native imaging orientation, the resulting image may suffer from reduced resolution and clarity.

Compounding techniques, which combine multiple adjacent slices to form a single 2D image, have been developed to address some of these issues. While these methods can improve signal-to-noise ratio and enhance certain features, they often introduce new problems such as blurring, ghosting artifacts, and loss of spatial resolution. Thus, when performing compounding, there is a trade-off between noise reduction and image sharpness.

Therefore, there is a present need for improved image processing techniques that reduce the noise present in 2D slices generated from 3D medical image data, whilst minimizing the presence of blur.

### SUMMARY OF INVENTION

The invention is defined by the claims.

According to an aspect of the present disclosure, a method for processing pixels of interest of a 3D volume of an anatomical structure to generate a 2D image of the anatomical structure is provided. The method comprises: for each of the pixels of interest, identifying one or more compound pixels in the vicinity of the respective pixel in the 3D volume based on one or more features of the anatomical structure included in the 3D volume; determining an updated value of the respective pixel based on values of the compound pixels. The method further comprises generating the 2D image based on the updated values of the pixels of interest.

This method allows for adaptive compounding of pixels in the 3D volume, taking into account the underlying anatomical features. By identifying compound pixels based on the anatomical structure, the method can reduce blurring and ghosting artifacts typically associated with standard compounding techniques, while still benefiting from noise reduction.

To be clear, the present invention provides concepts for generating high-quality two-dimensional (2D) images from three-dimensional (3D) volumetric data of anatomical structures using adaptive compounding techniques. The invention addresses the challenges associated with standard slice extraction and compounding methods, which often result in blurring, ghosting artifacts, and loss of spatial resolution.

At the core of the invention is an adaptive compounding approach that takes into account the underlying features of the anatomical structure when processing pixels to generate a 2D image. For each pixel of interest in the desired 2D image plane, the method identifies compound pixels in the 3D volume based on local anatomical features. This approach allows for a more intelligent selection of pixels to be combined, reducing artifacts typically associated with standard compounding techniques.

In existing techniques, pixels in the vicinity of each pixel of interest in the 3D volume are identified based on a compounding direction, for example. These pixels are then used to enhance the initial 2D image slice, to reduce the presence of artifacts, decrease noise, etc. This compounding direction may be the same for each of the pixels, and may not take into account the underlying anatomical features. Accordingly, the resultant image may include excessive levels of blur, particularly in regions on the edge/boundary of certain anatomical features. Accordingly, the present invention proposes to identify compounding pixels for updating values of the initial slice based on the underlying shape of the anatomical features.

The proposed method offers several advantages over existing techniques. It provides improved image quality by reducing artifacts and enhancing feature preservation, potentially leading to more accurate diagnoses and improved patient care. The adaptive nature of the compounding process allows for better visualization of complex anatomical structures, which is particularly beneficial in cases where standard slice extraction methods may struggle to provide clear images. Additionally, the method is flexible and can be applied to various 3D imaging modalities, making it a versatile tool for medical imaging professionals across different specialties.

In some embodiments, the method may further comprise, for each of the pixels of interest, determining a compounding direction associated with the respective pixel, the compounding direction based, at least in part, on a shape of the one or more features included in the 3D volume, wherein identifying the compound pixels is based on the compounding direction.

By determining a compounding direction based on the shape of anatomical features, the method can better align the compounding process with the underlying structures, reducing artifacts and improving image quality. The invention thus further introduces the concept of adaptive compounding directions. Rather than using a fixed compounding direction for all pixels, the method determines a compounding direction for each pixel based on the shape of nearby anatomical features. This adaptive approach allows the compounding process to better align with the underlying structures, significantly reducing blurring and ghosting artifacts while preserving important details.

Existing compounding techniques that use a plurality of neighboring slices of the 3D volume to enhance the slice of interest may be modified in a relatively straightforward manner. That is, by simply augmenting the compounding direction for each pixel based on the local anatomical features in the vicinity of the pixel, an improved compounding result may be achieved.

The method may further comprise, for each of the pixels of interest, identifying a gradient of a feature in the 3D volume in the vicinity of the respective pixel. Determining the compounding direction in this case is further based on the identified gradient.

Incorporating the gradient information allows the method to adapt the compounding direction to local changes in image intensity, providing more accurate alignment with anatomical structures and potentially improving edge preservation in the resulting 2D image.

In some embodiments, the method may further comprise, for each of the pixels of interest, identifying a projection direction at the location of the respective pixel. Determining the compounding direction in this case is further based on the identified projection direction.

By considering both the projection direction and the local feature orientation, the method can achieve a balance between maintaining the desired viewing perspective and adapting to the underlying anatomical structures.

Specifically, the projection direction may be a direction perpendicular to the image plane of the 2D image at the location of the respective pixel. This ensures that the compounding process takes into account the desired viewing plane while still allowing for local adaptations based on anatomical features. Indeed, typical compounding techniques are based on a compounding direction perpendicular to the image plane, and therefore this provides for a straightforward adaptation of existing techniques.

The method of identifying the compound pixels based on the compounding direction may comprise: identifying a ray in the 3D volume passing through the location of the respective pixel in the compounding direction, the ray having a predetermined length; identifying pixels in the 3D volume incident to the ray; and identifying the compound pixels based on the identified pixels incident to the ray.

This approach provides a systematic way to identify compound pixels along the adaptive compounding direction, allowing for efficient implementation of the method.

In addition, the method may further comprise determining the length of the ray based on the compounding direction and a gradient of an identified feature at the location of the respective pixel.

Adapting the ray length based on local feature characteristics allows for variable compounding thickness, potentially improving the method's ability to preserve fine details while still reducing noise in more homogeneous regions. Indeed, with a larger ray length, the more noise and artifacts may be reduced. Nevertheless, a large ray length may result in additional blur, even when anatomical features are considered. Therefore, an appropriate ray length must be identified.

The compounding direction of each respective pixel may be further based on the compounding directions of adjacent pixels of interest. This normalization results in spatial coherence of compounding directions can help ensure smoother transitions between regions and reduce potential artifacts caused by abrupt changes in compounding direction. Nevertheless, it may be desirable to minimize this normalization, particularly around sharp anatomical feature borders, to ensure minimal blur. This will, of course, depend on the imaged anatomy, plane of the 2D image, and user preferences.

In some embodiments, the method may further comprise a step of processing the compounding directions of each of the pixels with a regularization operator, to determine regularized compounding directions. This may help to further reduce blurring and ghosting artifacts and improve the alignment of the compounding directions with the underlying features of the anatomical structure.

In some embodiments, the step of determining the updated value of the respective pixel may comprise processing, with a compound operator, the value of the respective pixel and the values of the compound pixels. This allows for flexibility in how the compound pixels are combined, enabling the method to be adapted for different imaging modalities or specific clinical needs.

In particular, the compound operator may be any one of an averaging operator, a weighted averaging operator, a minimum selector, or a maximum selector. Each of these operators can provide different benefits, such as noise reduction with averaging, edge preservation with maximum selection, or contrast enhancement with minimum selection, allowing the method to be tailored to specific imaging requirements.

The method may further comprise, for each of the pixels of interest, identifying a location of the respective pixel in the 3D volume. This step ensures accurate mapping between the 2D image and the original 3D volume, which is crucial for maintaining spatial relationships and enabling potential further analysis or processing.

The method may further comprise a preceding step of identifying the pixels of interest of the 3D medical image, each of the pixels of interest in a same image plane of the 3D medical image, and optionally wherein the image plane is based on a received indication of a slice of the 3D volume. This allows for user-defined selection of the region of interest within the 3D volume, providing flexibility in generating 2D images from specific planes or slices of interest.

According to another aspect of the present disclosure, a computer program comprising computer program code means adapted, when said computer program is run on a computer, to implement the method described above is provided.

According to another aspect of the present disclosure, a system for processing pixels of interest of a 3D volume of an anatomical structure to generate a 2D image of the anatomical structure is provided. The system comprises: an analysis unit configured, for each of the pixels of interest, to identify one or more compound pixels in the vicinity of the respective pixel in the 3D volume based on one or more features of the anatomical structure included in the 3D volume, and determine an updated value of the respective pixel based on values of the compound pixels; and a graphics unit configured to generate the 2D image based on the updated values of the pixels of interest.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for processing 3D volumetric data to generate 2D images, according to aspects of the present disclosure;
Fig. 2 depicts a system for processing a 3D volume to generate a 2D image, according to an embodiment;
Fig. 3 shows a system for processing a 3D volume to generate a 2D image with adaptive compounding, according to aspects of the present disclosure;
Fig. 4 presents a flowchart for processing pixels of a 3D medical image to generate a 2D image, according to an embodiment;
Fig. 5 illustrates a diagram representing a system for adaptive compounding in image processing, according to aspects of the present disclosure;
Fig. 6 depicts a block diagram of a system for processing 3D volume data to generate 2D images, according to an embodiment; and
Fig. 7 is a simplified block diagram of a computer within which one or more parts of an embodiment may be employed.

### DETAILED DESCRIPTION OF EMBODIMENTS

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to provide an advantage.

The present disclosure provides concepts for processing pixels of interest of a 3D volume of an anatomical structure to generate a 2D image of the anatomical structure. The method includes, for each of the pixels of interest: identifying one or more compound pixels in the vicinity of the respective pixel in the 3D volume based on one or more features of the anatomical structure included in the 3D volume; and determining an updated value of the respective pixel based on values of the compound pixels. The method further includes generating the 2D image based on the updated values of the pixels of interest. The method allows for adaptive compounding based on the shape of the anatomical structure included in the 3D volume, such that blurring and ghosting artifacts are reduced, while maintaining the benefits of compounding techniques (e.g., noise reduction).

The present disclosure provides a method and system for generating two-dimensional (2D) images from three-dimensional (3D) volumetric data of anatomical structures using an adaptive compounding technique. This technique is designed to address the challenges associated with standard slice extraction and compounding methods, which often result in blurring, ghosting artifacts, and loss of spatial resolution.

The adaptive compounding technique disclosed herein takes into account the underlying features of the anatomical structure when processing pixels to generate a 2D image. For each pixel of interest in the desired 2D image plane, the method identifies compound pixels in the 3D volume based on local anatomical features (i.e., the shape of anatomical features in the 3D volume that are in the vicinity of the respective pixel). This approach allows for a more intelligent selection of pixels to be combined, reducing artifacts typically associated with standard compounding techniques.

Furthermore, the present disclosure introduces the concept of adaptive compounding directions. Rather than using a fixed compounding direction for all pixels, the method determines a compounding direction for each pixel based on the shape of nearby anatomical features. This adaptive approach allows the compounding process to better align with the underlying structures, significantly reducing blurring and ghosting artifacts while preserving important details.

The proposed method and system offer several potential advantages. They provide improved image quality by reducing artifacts and enhancing feature preservation, potentially leading to more accurate diagnoses and improved patient care. The adaptive nature of the compounding process allows for better visualization of complex anatomical structures, which is particularly beneficial in cases where standard slice extraction methods may struggle to provide clear images. Additionally, the method and system are flexible and can be applied to various 3D imaging modalities, making them versatile tools for medical imaging professionals across different specialties.

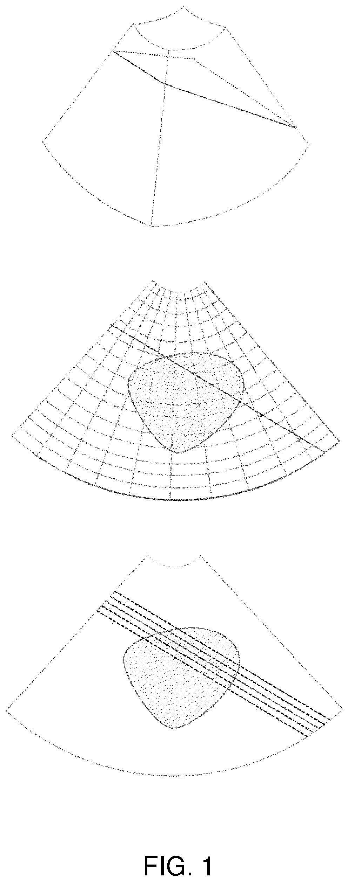

To best understand the present invention, it is helpful to first understand existing techniques for slice extraction from 3D volumes and associated compounding techniques. This is represented in simplified form in Fig. 1.

Visualization packages for 3D imaging modalities (Ultrasound, CT, MR, etc.) usually include a slice extraction module to display 2D images from a 3D acquisition volume. When 2D slices do not coincide with native imaging orientations (e.g. axial CT slices), they are usually referred to as Multi-Planar Reconstructions (MPR). A slice of an MPR in 3D ultrasound is represented by the top image Fig. 1. This is represented in 2D in the middle image in Fig. 1, in which the shape of an anatomical feature is also represented. It is worth noting that the 2D slice may not be a flat plane, but may be curved or have another shape, for example to follow the shape of an anatomy.

In order to improve the perceived quality of MPRs, compounding techniques are often used. In essence, adjacent slices of the 3D volume to the 2D slice of interest are taken, and the values of the pixels in those slices used to generate a single 2D slice. Examples for processing of the values include maximum intensity projection (MIP) or simple averaging, also referred to as "thick slice" imaging. Slice thickness (or the number of adjacent slices used) is a typical parameter to control the balance of imaging performance in terms of sensitivity, contrast, noise, and resolution. A trade-off is to be found between beneficial de-noising and detrimental blurring, resulting in loss of spatial resolution introduced by compounding, which this invention attempts to mitigate.

This process is represented graphically in the bottom image Fig. 1. Specifically, the middle line corresponding to the 2D slice of interest. The dotted lines represent the other slices used to improve the 2D slice of interest. These slices are combined to form a single MPR.
In one implementation, MPR compounding can be implemented by ray casting. This can be summarized by the following pseudo-code:

| | |
|---|---|
| | Given thickness T and plane direction N |
| | For each Pixel XY of the output image |
| 1. | Find 3D position P in the input volume that corresponds to Pixel XY |
| 2. | Create a ray segment of origin P, length T and direction N |
| 3. | Calculate compound operator on volume samples interpolated along ray |
| 4. | Write compound result as output value at Pixel XY |
| End | |

Any compound operator that combines multiple samples can be substituted in Step 3. For instance, maximum intensity projection (MIP) is obtained if the compound operator is chosen as a max operator. The aggregated result in Step 4 will then correspond to the maximum brightness of all voxels sampled and interpolated along the ray. MIP is a popular choice to increase sensitivity ensuring features from neighboring planes are present. If an average() operator is chosen, the technique becomes equivalent to a "thick slice" or "slab" of thickness T. Compounding by averaging has the advantage of de-noising the output MPR image and increasing the signal-to-noise ratio (SNR). Depending on the application, other choices of operator include min(), median(), or virtually any one-dimensional operator.

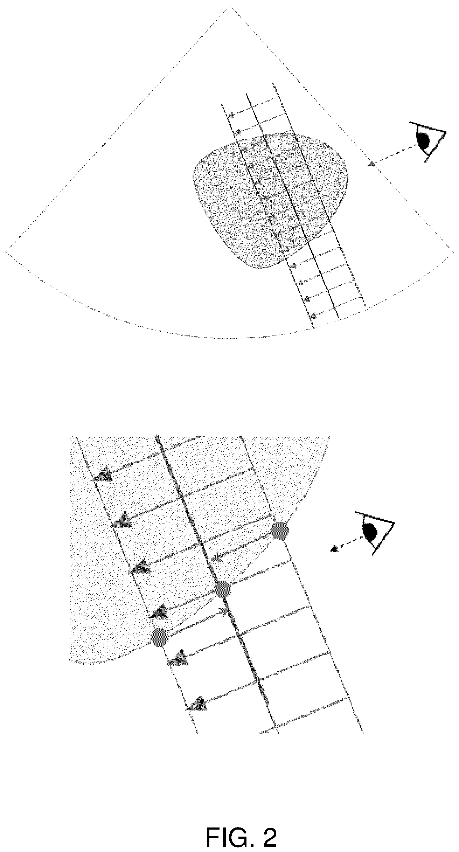

Problems addressed by embodiments of the invention are include the negative consequences of increasing the thickness T. Due to the fact that the compounding direction is not necessarily well aligned with the underlying image features in 3D, ghosting artefacts appear on strong edges. This can be seen in Fig. 2, with the top image presenting the slice through the anatomy, and the compounding direction (the arrows in the figure). As best seen in the bottom image, when the compounding direction is simply perpendicular to the image plane and the pixels are compounded on the edge of the anatomical feature, some of the compound pixels will include the bulk part of the anatomical feature, whilst other compound pixels will include adjacent material. In other words, parts of the 3D volume corresponding to the edge of the anatomical feature will be used for compounding in a plurality of adjacent pixels of interest.

These ghosting artefacts are unwanted repetitions of image features. As edges of the same object in each of the compounded planes do not spatially coincide, edges of the same object will be projected onto different locations in the compounded plane, resulting in blurring, ghosting, and a loss of spatial resolution. Accordingly, it is a common approach to reduce the thickness to achieve an acceptable tradeoff between de-noising and blurring. Furthermore, ghosting artefacts in MIP can be mitigated by using a denser sampling density along the ray, which tends to merge repeated edges together. Unfortunately, this still results in a loss of resolution and increases computational complexity.

Disclosed embodiments of the invention improve upon the trade-off between de-noising and blurring by reducing the amount of blur generated by compounding, without needing to decrease the thickness T. After observing that blurring is introduced in regions where image feature orientation and compounding direction are not well aligned, it was realised that identification of compounding pixels for each pixel of interest (i.e., pixel in a target image plane) can be improved by taking into account local anatomical features. For example, in some embodiments the compounding direction is locally modified based on anatomical features in the vicinity of the pixel.

One embodiment of the invention may be best understood by adaptation of the pseudo-code above. The primary difference with the standard compounding technique described in the above pseudo-code is an additional step to calculate, for each pixel of the output image, a spatially-varying version of the compounding direction. Thus, the pseudo-code according to an embodiment is as follows:

| | |
|---|---|
| | Given thickness T and plane direction N |
| | For each Pixel XY of the output image |
| 1. | Find 3D position P in the input volume that corresponds to Pixel XY |
| 2. | Calculate a locally modified direction M as a function of P and N |
| 3. | Create a ray of origin P, length T and direction M(P,N) |
| 4. | Calculate compound operator on volume samples interpolated along ray |
| 5. | Write compound result as output value at Pixel XY |
| | End |

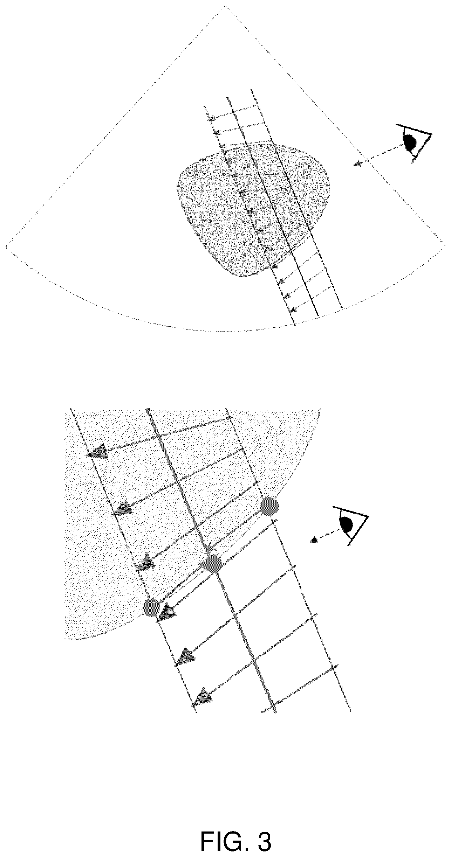

Compared to standard compounding, achieved with a constant direction defined by the normal to the plane being reconstructed, the compounding direction is now locally adapted based on the underlying anatomical object shape. This shape-adaptive compounding process can be seen in Fig. 3, in which the arrows, which represent compounding directions, now vary between pixels of interest to better align with 3D image features. By improving this alignment, image features from the same object on neighboring planes now project onto the same location in the image, thereby reducing the spatial footprint of that particular feature on the compounded image. Thus, fewer ghosting artefacts may be present.

Furthermore, and although not depicted, it is also proposed that the ray may not be a straight line but may have a curved trajectory following the underlying anatomical features. That is, the ray may be parallel with, and incident to, the determined locally modified/compounding direction at the position P but may curve based on underlying features also. This means that the identified samples interpolated along the ray may be more likely to relate to the same anatomical feature. Thus, the straight lines depicted in Fig. 3 may be curved based on the underlying anatomical features.

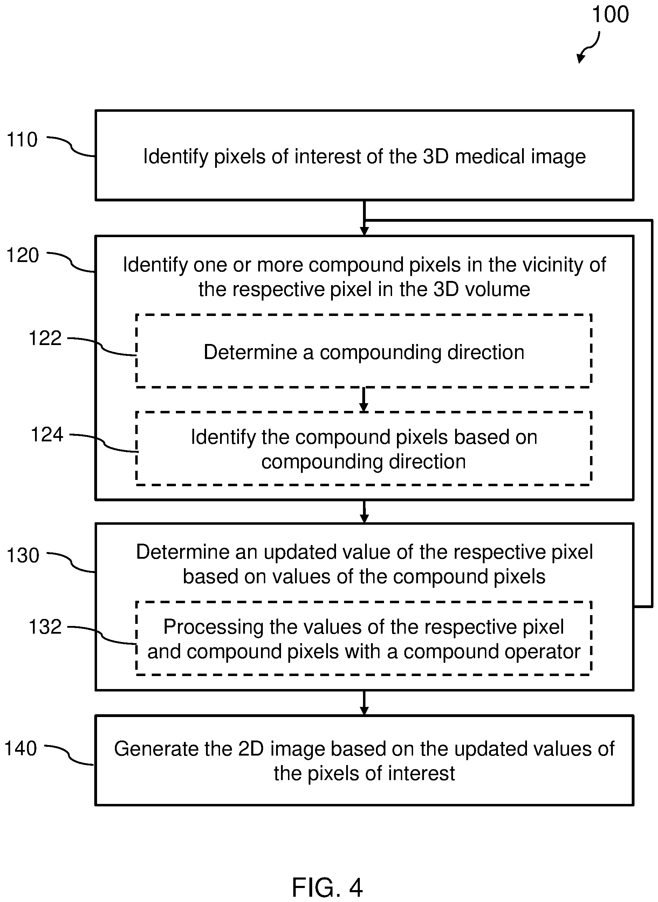

Referring to Fig. 4, a flowchart for a method of processing pixels of a 3D medical image to generate a 2D image is illustrated. Specifically, the method processes pixels of interest of a 3D volume of an anatomical structure to generate a 2D image of the anatomical structure. The method may thus produce a slice of 3D medical data to provide a 2D image that is easier to visualize and potentially simpler for a clinician to interpret.

The pixels of interest may all correspond to a same image plane. That is, each of the pixels of interest may lie on a same image plane. The image plane may be a flat image plane, or may be curved, for example to match the curve on an anatomical feature such as a spine, a rib, or a vein.

The 3D volume may be acquired using any medical imaging modality, such as MRI, CT, ultrasound, etc. The processing may occur directly subsequent to acquiring the 3D volume, for example in real time, or may be part of a post-processing operation performed during analysis and interpretation of the 3D volume acquisition.

In step 110, the method begins by identifying pixels of interest in the 3D medical image. Each of the pixels of interest are in a same image plane of the 3D medical image. That is, each of the pixels of interest may correspond to a slice of the 3D medical image. In some aspects, the pixels of interest may be those that correspond to a particular feature or region of interest within the anatomical structure. For example, the pixels of interest may be those that correspond to a tumor or other abnormality in a slice within the anatomical structure.

In some embodiments, the image plane of the pixels of interest is based on a received indication of a slice of the 3D volume. That is, a user may indicate a location of the 2D image to be generated, and the pixels of interest identified within the 3D volume based on this indication. Therefore, for each of the pixels of interest, a location of the respective pixel in the 3D volume may be identified.

For each of the identified pixels of interest, the method involves identifying one or more compound pixels in the vicinity of the respective pixel in the 3D volume and determining an updated value of the respective pixel. This is represented by the loop between steps 130 and 120. Nevertheless, it is worth noting that these steps may be completed in different orders. For example, step 120 may be completed for each pixel of interest before step 130 is completed for each pixel of interest.

In any case, in step 120, one or more compound pixels in the vicinity of the respective pixel in the 3D volume are identified based on one or more features of the anatomical structure included in the 3D volume. That is one or more pixels whose values will be used to adapt the value of the respective pixel are identified. This identification is based on one or more features of the anatomical structure included in the 3D volume, such as the shape of features of the anatomical structure in the vicinity of the respective pixel, an orientation of the feature, a size of the feature, etc. As a result, the compound pixels may be less likely to introduce ghosting artifacts and blur.

More particularly, step 120 may comprise sub-step 122 in which a compounding direction associated with the respective pixel is determined. To be clear, the compounding direction is based, at least in part, on a shape and/or orientation of the one or more features included in the 3D volume.

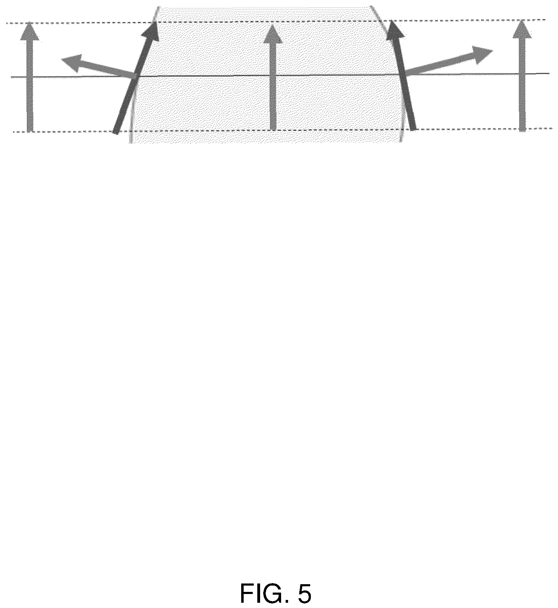

Different factors and methods for determining the compounding direction will be described in more detail below in reference to Fig. 5.

In sub-step 124, the compound pixels for the respective pixel are identified based on the compounding direction. To be specific, pixels in the 3D volume that lie in the compounding direction through the respective pixel may be identified as compound pixels. In some cases, the compound pixels may be those that are located along a ray in the 3D volume that passes through the location of the respective pixel in the compounding direction.

To this end, identifying the compound pixels based on the compounding direction may comprise identifying a ray in the 3D volume passing through the location of the respective pixel in the compounding direction. The ray may have a predetermined length, such as the thickness T described above, which may depend on the extent of de-noising that is desired. Pixels are then identified in the 3D volume incident to the ray. Thus, each pixel that lies in the compounding direction from the respective pixel will be identified. The compound pixels are then identified based on the identified pixels incident to the ray. Indeed, not all the pixels may be identified as compound pixels, and/or some additional pixels may be identified.

In further embodiments, the identified ray may not be a straight ray. That is, the identified ray may be curved based on underlying anatomical features. The identified curved ray may be parallel with, and incident, to the compounding direction through the location of the respective pixel. However, as the ray is followed away from the location of the respective pixel, the ray may deviate from the compounding direction based on underlying anatomical features. Thus, identified compounding pixels may more likely correspond to a same or similar anatomical feature, particularly at feature boundaries.

For instance, the volume could be traversed by following a ray starting at the position of the respective pixel and along the same compounding direction, with a given step size. In this case, at each step the compounding direction would be updated as a function of the underlying 3D features encountered along the ray. Thus, the straight ray would become curved, potentially improving preservation of features at the expense of additional algorithmic complexity.

In some cases, the length of the ray may be determined based on the compounding direction and a gradient of an identified feature at the location (i.e., in the vicinity) of the respective pixel. This adaptive ray length may reduce the amount of blur. For example, if the gradient/shape of the feature indicates that the respective pixel corresponds to the corner of a feature, then the ray length may be short. In contrast, if it is determined that the respective pixel is in the center of a large homogenous feature, then the length of the ray may be elongated to reduce noise.

In step 130, an updated value is determined for each respective pixel based on the values of the compound pixels. For example, the values of the compound pixels may be used to verify and/or modify the value of the respective pixel to determine the updated pixel.

In some embodiments, the method may comprise step 132 in which the updated value of the respective pixel is determined by processing the values of the respective pixel and compound pixels with a compound operator. The compound operator may be any one of an averaging operator, a weighted averaging operator, a minimum selector, a maximum selector, or any other 2D operator. In some aspects, the compound operator may be chosen based on the desired image quality or the specific application of the 2D image.

Finally, in step 140, the method generates the 2D image based on the updated values of the pixels of interest. This may simply involve combining the values of the pixels of interest to provide the 2D image. In other cases, post-processing may be performed to smooth any abrupt changes between adjacent pixels that have arisen due to the method. In any case, the updated values of the pixels of interest provide a more accurate representation of the underlying anatomical structure, reducing blurring and ghosting artifacts and enhancing the quality of the 2D image.

In some aspects, the adaptive compounding technique may be applied to multi-channel images. Multi-channel images are those that contain multiple images or channels for a 3D acquisition, such as T1 and T2 in MRI, or B-mode and Doppler in 3D Ultrasound. The adaptive compounding technique can be used on each channel independently, allowing for the processing of each channel to be tailored to its specific characteristics. Alternatively, one of the channels can be chosen to drive the calculation of shape-adaptive compounding directions for other channels. This alternative can be beneficial to ensure that directions are consistent for all channels, or if one of the channels has better definition of the relevant image features.

In some cases, the adaptive compounding technique may be applied to 2D Ultrasound imaging in the context of Elevation Angular Compounding (EAC). Elevation Angular Compounding is a method to reduce speckle by combining multiple 2D planes that have been acquired at small angular elevation intervals around a central slice. Since the same three-dimensional objects are seen from multiple consecutive planes, the principle of per-pixel adaptation of the compounding direction to follow the underlying three-dimensional object shape readily applies and should provide similar benefits. This application of the adaptive compounding technique can lead to improved image quality by reducing speckle and enhancing feature preservation.

In other aspects, the adaptive compounding technique may be applied to other 3D imaging modalities. These modalities may include, but are not limited to, Computed Tomography (CT), Cone-Beam CT (CB-CT), and Magnetic Resonance Imaging (MRI). The adaptive compounding technique can be readily applied to these modalities, providing improved image quality by reducing blurring and ghosting artifacts, and enhancing feature preservation. This makes the adaptive compounding technique a versatile tool for medical imaging professionals across different specialties.

Referring to Fig. 5, the diagram represents the adaptation of the compounding direction based (at least in part) on a shape of the one or more features included in the 3D volume. As can be seen, the compounding direction for pixels near the edge of the anatomical feature (represented by the shaded area) are changed to be tangential to the outline of the shape. In contrast, the compounding direction for pixels far outside the anatomical feature or within the anatomical feature, the compounding direction remains perpendicular to the image plane (represented by the solid horizontal line).

More generally, for each of the pixels of interest, a gradient of a feature in the 3D volume in the vicinity of the respective pixel may be identified. This gradient may be found by segmenting anatomical features in the 3D volume and generating a gradient map, for example. Accordingly, the compounding direction for the respective pixel is further based on the identified gradient.

Of course, determining the compounding direction for each of the pixels of interest may also comprise identifying a projection direction at the location of the respective pixel. The projection direction may be the direction used for a typical compounding operation. This is usually substantially perpendicular to the image plane at the location of the respective pixel. It is worth noting that the projection direction may vary for each pixel in the case that the 2D image plane is non-flat (e.g., curved). However, if the 2D image plane is flat, then the projection direction may be determined only once to cover all of the pixels of interest.

Accordingly, determining the compounding direction further based on the identified projection direction means that the identified projection direction is modified based on (the shape and/or orientation of) one or more features in the 3D volume.

In one embodiment, modifying the compound direction is based on the orthogonal projection of the plane normal vector on the plane perpendicular to the image gradient of an anatomical feature in the vicinity of the pixel of interest. The image gradient vector of the original volume may be obtained by a first-order difference operator applied to the original 3D volume that is pre-convolved with a Gaussian kernel.

The compounding direction may be the smallest modification of the identified projection direction that results in a vector that is tangential to an iso-surface of an anatomical feature in the 3D volume. As a result, the generation of the updated value (e.g., as a result of aggregation) is performed in a direction of minimal brightness change/variation. In other words, it is the smallest modification of the compounding direction that minimizes blur.

In some cases, the method may utilize an external shape finder or 3D segmentation to drive the calculation of optimal compounding directions. The external shape finder or 3D segmentation may provide a model of the anatomical structure, which can be used to guide the compounding process. For example, if a 3D mesh of the segmentation is available, the compounding direction may be orthogonal to the mesh surface in areas where it intersects the plane being reconstructed. Away from the segmentation boundary, directions will need to be propagated, for instance by diffusion.

In some aspects, the method may use a volumetric Euclidean distance map to the segmentation boundary to drive compounding. The distance map may provide a measure of the distance from each pixel in the 3D volume to the nearest point on the segmentation boundary. This information can be used to adjust the compounding direction, such that it is more aligned with the underlying structure of the anatomical feature. For instance, the compounding direction may be adjusted to be more parallel to the gradient of the distance map, thereby reducing blurring and ghosting artifacts in the resulting 2D image.

In some cases, the method may use a combination of these techniques to optimize the compounding directions. For example, the method may first use an external shape finder or 3D segmentation to determine an initial set of compounding directions, then refine these directions using a volumetric Euclidean distance map. This approach may provide a more accurate alignment of the compounding directions with the underlying features of the anatomical structure, thereby further improving the quality of the resulting 2D image.

Furthermore, the compounding direction of each respective pixel may be further based on the compounding direction of adjacent pixels of interest. By allowing the compounding direction to be based on the compounding directions of adjacent pixels, this may avoid abrupt changes in the compounding direction and thus the potential occurrence of artifacts.

In some cases, the compounding directions of each of the pixels may be processed with a regularization operator to determine regularized compounding directions. This additional step may help to further reduce blurring and ghosting artifacts and improve the alignment of the compounding directions with the underlying features of the anatomical structure. In some aspects, the regularization operator may be a mathematical function or algorithm that smooths or regularizes the compounding directions, thereby reducing abrupt changes in direction that could lead to artifacts in the resulting 2D image.

Nevertheless, in some cases, the compounding direction may remain unnormalized to offer additional shape adaptiveness. By keeping the vector unnormalized, this may allow the compounding thickness to better depend on the angle between the image feature orientation and the compounding direction at each pixel. This adaptive behavior can automatically adjust the local thickness according to how favorable the underlying image feature orientation is. For instance, if an image feature 3D orientation is perpendicular to the compounding direction, it should not be compounded at all because any compounding will create some blur. This intuitive adaptive behavior can be obtained with the system by keeping the compounding direction unnormalized. This approach can further enhance the quality of the resulting 2D image by reducing blurring and ghosting artifacts and preserving important details of the anatomical structure.

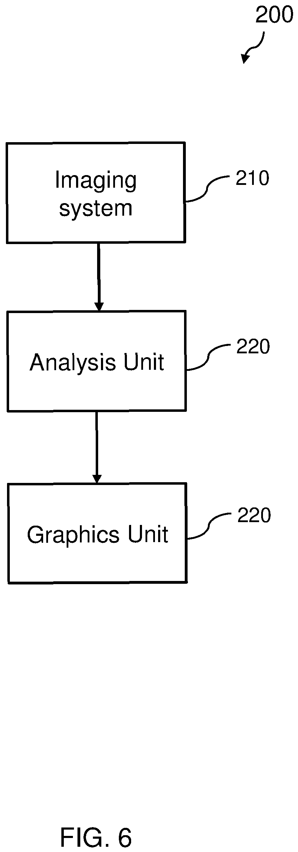

Referring to Fig. 6, a system 200 for processing pixels of interest of a 3D volume of an anatomical structure to generate a 2D image of the anatomical structure is illustrated. The system 200 comprises three main components: an imaging system 210, an analysis unit 220, and a graphics unit 230.

The imaging system 210 is serves as the input source for the system 200. This component may acquire or provide the 3D volume data of an anatomical structure. In some aspects, the imaging system 210 may be a medical imaging device, such as an ultrasound machine, a computed tomography (CT) scanner, a magnetic resonance imaging (MRI) machine, or any other device capable of acquiring 3D volumetric data of an anatomical structure.

Connected to the imaging system 210 is the analysis unit 220. This unit receives data from the imaging system 210 and performs processing operations on the 3D volume data. The analysis unit 220 may be a computer, a server, a dedicated hardware device, or any other device capable of processing 3D volumetric data. In some cases, the analysis unit 220 may include one or more processors and memory for storing and executing software instructions.

For each pixel of interest in the 3D volume, the analysis unit 220 is configured to identify one or more compound pixels in the vicinity of the respective pixel based on one or more features of the anatomical structure included in the 3D volume. The compound pixels may be identified based on their spatial relationship to the respective pixel and the features of the anatomical structure. In some aspects, the features of the anatomical structure may include intensity values, texture, shape, or other image properties.

To be clear, the compound pixels may be identified by the analysis unit using any method or combination thereof, disclosed herein.

The analysis unit 220 is also configured to determine an updated value of the respective pixel based on values of the compound pixels. This determination may involve processing the values of the respective pixel and compound pixels with a compound operator. The compound operator may be an averaging operator, a weighted averaging operator, a minimum selector, or a maximum selector, among others.

The final component in the system 200 is the graphics unit 230, connected to the analysis unit 220. This unit receives processed data from the analysis unit 220 and is responsible for generating the 2D image output based on the updated pixel values. The graphics unit 230 may incorporate a display device, a printer, a storage device, or any other device capable of generating or storing 2D images.

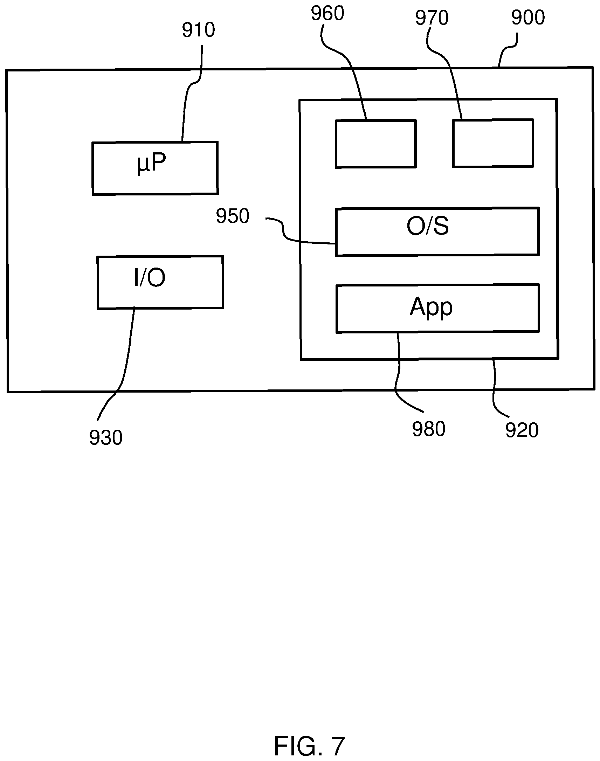

Fig. 7 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc.

Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to provide an advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A method (100) for processing pixels of interest of a 3D volume of an anatomical structure to generate a 2D image of the anatomical structure, the method comprising:
for each of the pixels of interest:
identifying (120) one or more compound pixels in the vicinity of the respective pixel in the 3D volume based on one or more features of the anatomical structure included in the 3D volume; and
determining (130) an updated value of the respective pixel based on values of the compound pixels; and
generating (140) the 2D image based on the updated values of the pixels of interest.

2. The method of claim 1, further comprising, for each of the pixels of interest, determining (122) a compounding direction associated with the respective pixel, the compounding direction based, at least in part, on a shape of the one or more features included in the 3D volume, and
wherein identifying (120) the compound pixels is based on the compounding direction.

3. The method of claim 2, further comprising, for each of the pixels of interest, identifying a gradient of a feature in the 3D volume in the vicinity of the respective pixel, and wherein
determining (122) the compounding direction is further based on the identified gradient.

4. The method of claim 2 or 3, further comprising, for each of the pixels of interest, identifying a projection direction at the location of the respective pixel, and
wherein determining (122) the compounding direction is further based on the identified projection direction.

5. The method of claim 4, wherein the projection direction is a direction perpendicular to the image plane of the 2D image at the location of the respective pixel.

6. The method of any of claims 2-5, wherein identifying (124) the compound pixels based on the compounding direction comprises:
identifying a ray in the 3D volume passing through the location of the respective pixel in the compounding direction, the ray having a predetermined length;
identifying pixels in the 3D volume incident to the ray; and
dentifying the compound pixels based on the identified pixels incident to the ray.

7. The method of claim 6, further comprising determining the length of the ray based on the compounding direction and a gradient of an identified feature at the location of the respective pixel.

8. The method of any of claims 2-7, wherein the compounding direction of each respective pixel is further based on the compounding direction of adjacent pixels of interest.

9. The method of claim 8, further comprising a step of processing the compounding directions of each of the pixels with a regularization operator, to determine regularized compounding directions.

10. The method of any of claims 1-9, wherein determining (130) the updated value of the respective pixel comprises processing (132), with a compound operator, the value of the respective pixel and the values of the compound pixels.

11. The method of claim 10, wherein the compound operator is any one of an averaging operator, a weighted averaging operator, a minimum selector, or a maximum selector.

12. The method of any of claims 1-11, further comprising, for each of the pixels of interest, identifying a location of the respective pixel in the 3D volume.

13. The method of any of claims 1-12, further comprising a preceding step of identifying (110) the pixels of interest of the 3D medical image, each of the pixels of interest in a same image plane of the 3D medical image, and optionally wherein the image plane is based on a received indication of a slice of the 3D volume.

14. A computer program comprising computer program code means adapted, when said computer program is run on a computer, to implement the method of any of claims 1-13.

15. A system (200) for processing pixels of interest of a 3D volume of an anatomical structure to generate a 2D image of the anatomical structure, the system comprising:
an analysis unit (220) configured, for each of the pixels of interest, to:
identify one or more compound pixels in the vicinity of the respective pixel in the 3D volume based on one or more features of the anatomical structure included in the 3D volume; and
determine an updated value of the respective pixel based on values of the compound pixels; and
a graphics unit (230) configured to generate the 2D image based on the updated values of the pixels of interest.
